# EUROPEAN PATENT APPLICATION

(11) **EP 1 839 661 A2**
(43) Date of publication of application: **03.10.2007**
(21) Application number: 07251317.9
(22) Date of filing: 28.03.2007
(51) Int. Cl.: A61K 31/4453, A61P 3/06, A61P 3/10, A61P 9/10, A61P 9/12

(54) **Pipecolic acid-containing antidiabetic compositions**

(30) Priority: 29.03.2006 JP 2006091147
(71) Applicant: Ajinomoto Co., Inc., Tokyo (JP)
(72) Inventor: Tagaki, Tomo, Kawasaki-shi, Kanagawa (JP); Nakano, Takashi, Kawasaki-shi, Kanagawa (JP); Ohta, Fumio, Kawasaki-shi, Kanagawa (JP); Miyazawa, Yuki, Kawasaki-shi, Kanagawa (JP); Satou, Hiroyuki, Kawasaki-shi, Kanagawa (JP)
(74) Representative: Nicholls, Kathryn Margaret

(57) **Abstract**

The present invention discloses a composition containing a component that is an in vivo intrinsic substance and widely-consumed. The present invention provides a composition containing pipecolic acid in its provided amount of 20mg/kg to 2000mg/kg of body weight per day. The composition of the present invention can be used, for example, in the treatment of diabetes.

## Description

The present invention relates to compositions containing pipecolic acid, and pharmaceutical compositions, foods, supplements and feeds containing the same.

Recent years, patients with diabetes have been rapidly increasing because of dietary habits from Western and dietaries high in calories; shortage of exercise due to motorization; and phenomenon of aging. According to the survey on the actual conditions regarding diabetes conducted in 2002 by the Ministry of Health, Labour and Welfare, it was indicated that "individuals with suspected diabetes" were up to about 7.4 million, and the total number thereof together with "individuals that cannot deny the possibility to have diabetes" was up to about 16.2 million. This condition is becoming a critical social and healthcare problem in taking measures against life-style related diseases (Non-patent Literature 1).

As therapeutic agents of diabetes, it is common to use sulfonylurea preparations that act as insulin secretagogue; α -glucosidase inhibitors that inhibit excess rise of blood glucose after meals; or, lately, thiazolidine preparations that improve insulin resistance. However, these medicinal synthetic preparations are neither easy nor convenient to obtain because prescription thereof is required. In addition to it, various side-effects may be accompanied by administration or dosing of the preparations (Non-patent Literatures 2 and 3). Therefore, naturally-derived ones easy to obtain and with side·effects as less as possible are needed as antidiabetic agents used in the treatment of diabetes. For example, indigestible dextrin and other natural extracts are paid attention (Non-patent Literature 4 and Patent Literatures 1 and 2). However, since most of them inhibit increase of the blood glucose by inhibiting glucose absorption, they do not directly improve the patient's physical condition. Besides it, they may induce adverse effects on digestive tracts such as diarrhea and constipation. Accordingly, it is desired to develop antidiabetic agents with side-effects as less as possible and those acting directly on the improvement of pathology.

It is reported that arterial sclerosis is induced by life-style related diseases that are called as metabolic syndromes such as diabetes (by high blood glucose or insulin resistance), hyperlipidemia and accumulation of visceral fats. It is also known that the progression of arterial sclerosis develops myocardial infarction or cerebral infarction. The deaths caused by these two diseases account for 31.0% of the total deaths of Japanese, and the percentage exceeds that of the deaths caused by cancer, which account for 28.5%. Thus, it is eagerly desired to develop antiarteriosclerotic agents for preventing and/or improving arterial sclerosis. For example, it is known that triterpene alcohol that is contained in rice bran and γ-orizanol that is a generic term used to refer to esters of ferulic acid of various phytosterols provide antiarteriosclerotic effects by decreasing lipids in the blood (Patent Literature 3). Further, it is also expected that extracts of Eucalyptus plants have antiarteriosclerotic effects by providing antiobesity effects (Patent Literature 4).

Pipecolic acid is one of the amino acids, which is biosynthesized from lysine in the mammal's body. It is known that pipecolic acid is also abundantly contained in various widely-consumed plants, especially *Phaseolus* sp (Non-patent Literatures 5, 6 and 7). The metabolic pathway thereof is almost the same as that of lysine, and it is reported that pipecolic acid is finally metabolized into CO₂ through TCA cycle (Non-patent Literature 8). As described above, pipecolic acid is an intrinsic substance and widely-consumed. And its metabolic pathway is similar to that of lysine. Thus, it is believed that it has a very high safety.

On the other hand, it is reported that L-isomer of pipecolic acid is reabsorbed in the kidney of humans and its blood concentration is maintained (Non-patent Literature 5). However, it is unclear whether pipecolic acid specifically relates to living organization as an intrinsic regulator. Besides, no finding has been known at all that this acid works on diabetes. Further, the quantity of pipecolic acid that can be taken from foods is 15mg/kg of body weight per day at, the highest estimate. Thus, it is easily estimated that its amount is clearly quantitatively less than that of the present invention.
[Patent Literature 1] Japanese Patent Unexamined Publication No. 2002-316938
[Patent Literature 2] Japanese Patent Unexamined Publication No. 2001-181194
[Patent Literature 3] Japanese Patent Unexamined Publication No. Sho 60-248611
[Patent Literature 4] Japanese Patent Unexamined Publication No. 2001-270833
[Non-patent Literature 1] Report of the survey on the actual conditions regarding diabetes Ministry of Health, Labour and Welfare, 2002
[Non-patent Literature 2] Acta anaesthesiologica Scandinavica, 47; 221-225, 2003
[Non-patent Literature 3] Diabetes, 52; 2249-2259, 2003
[Non-patent Literature 4] Journal of nutritional food, 6; 89-98, 2003
[Non-patent Literature 5] Clinica Chimica Acta, 287; 145-156, 1999
[Non-patent Literature 6] Journal of Chromatography A, 708; 131-141, 1995
[Non-patent Literature 7] Journal of agricultural and food chemistry, 34; 282-284, 1986
[Non-patent Literature 8] Biochimica et Biophysica Acta, 675; 411-415, 1981

The object of the present invention is to provide a composition containing a component that is an in vivo intrinsic substance and widely-consumed.

The inventors found that administration of pipecolic acid to normal rats, obese model rats and diabetic model rats improves their glucose tolerances and insulin resistances and decreases their neutral fats in the blood and accumulation of visceral fats. The present invention has been completed based on this finding.

Namely, the present invention provides a composition containing pipecolic acid in its provided amount of 20mg/kg to 2000mg/kg of body weight per day.

The present invention also provides a composition containing pipecolic acid for: inhibiting increase of the blood glucose level or decreasing the blood glucose level; enhancing insulin sensitivity or improving insulin resistance; inhibiting increase of lipids in the blood or decreasing lipids in the blood; inhibiting accumulation of visceral fats or decreasing accumulated visceral fats; or preventing, diminishing or treating diabetes, arterial sclerosis, obesity or high-blood pressure.

The present invention further provides a pharmaceutical composition containing the above composition.

The present invention additionally provides a food containing the above composition.

The present invention further additionally provides a supplement containing the above composition.

The present invention further additionally provides a feed which contains the above composition.

According to the present invention, it is possible to provide a composition that has low toxicity and can directly and effectively prevent, diminish or treat the above diseases or pathologies such as diabetes.

Embodiments of the invention are described below, by way of example, and with reference to the accompanying drawings, of which:

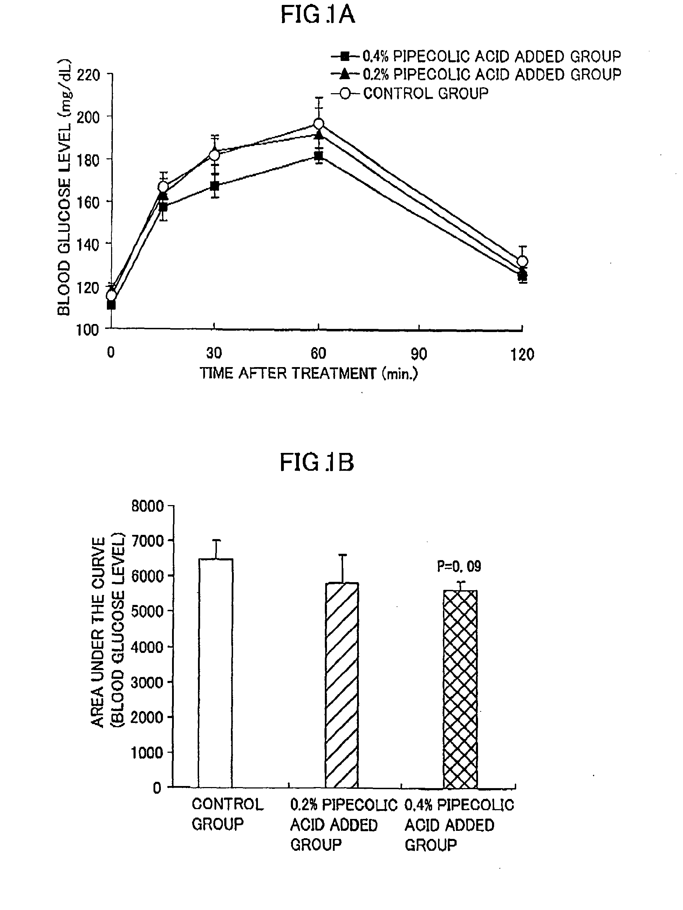

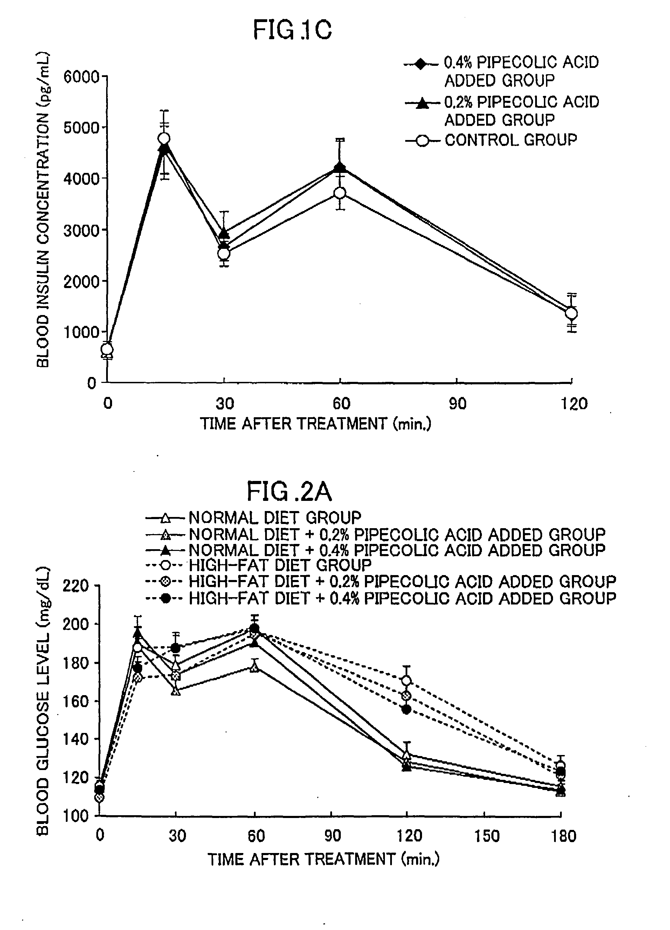

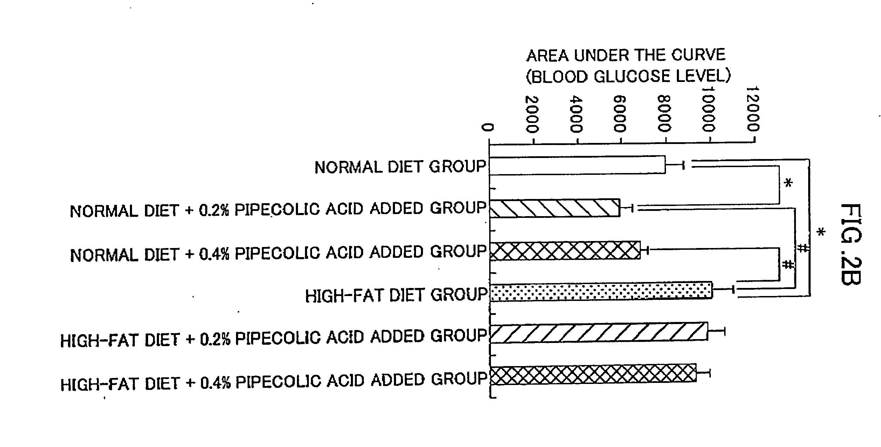

Figure 1 (Fig.1A to Fig.1C) shows diagrams that indicate results of the oral glucose tolerance test conducted in the test 1. Fig. 1A and Fig.1B indicate the blood glucose transitions after injection of glucose and the area under the blood glucose concentration time curve; and Fig.1C indicate transitions of the blood insulin concentration after injection of glucose. Value P in the diagram indicates a risk rate as compared with the control group.

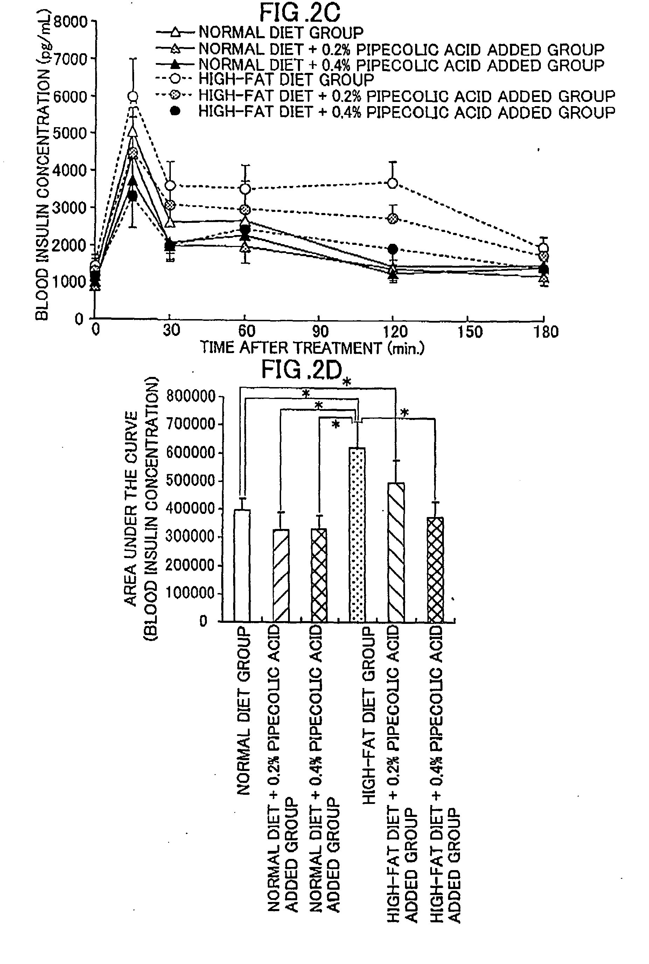

Figure 2 (Fig.2A, to Fig.2D) shows diagrams that indicate results of the oral glucose tolerance test conducted in the test 2. Fig.2A and Fig.2B indicate the blood glucose transitions after injection of glucose and the area under the blood glucose concentration time curve; and Fig.2C and Fig.2D indicate transitions of the blood insulin concentration after injection of glucose. Each * and # indicates that there is a significant difference between the corresponding groups of P<0.05, and P<0.01, respectively.

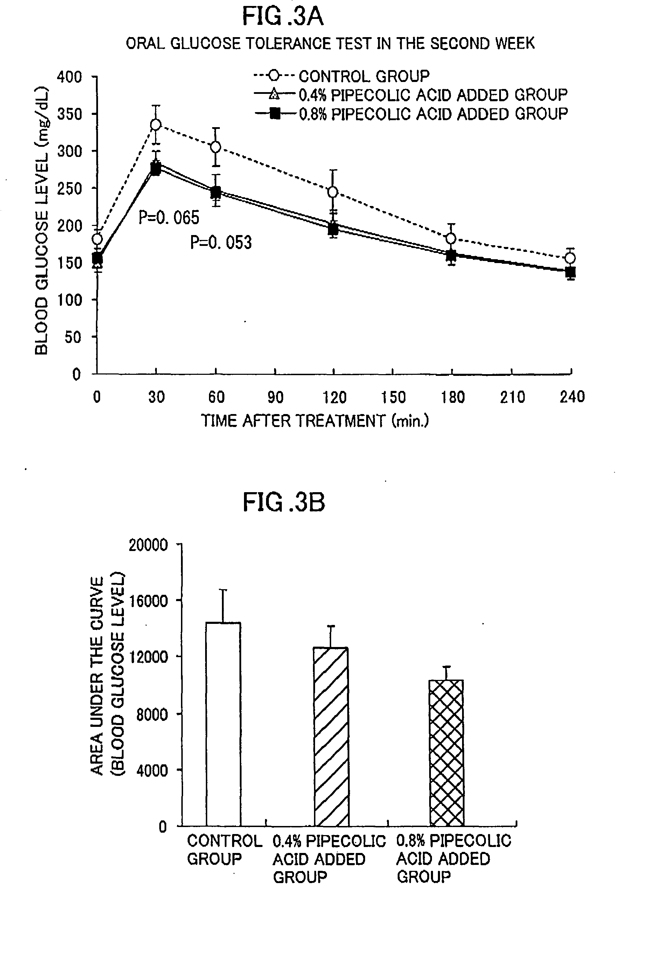

Figure 3A to Figure 3D shows diagrams that indicate results of the oral glucose tolerance test conducted in the test 3. Fig.3A to Fig.3D indicate transitions of the blood glucose and insulin concentration after injection of glucose at the second week after providing test diets and areas under the concentration time curve thereof. Each * and # indicates that there is a significant difference as compared with the control group at P<0.05, and P<0.01, respectively. Further, value P in the diagram indicates a risk rate as compared with the control group.

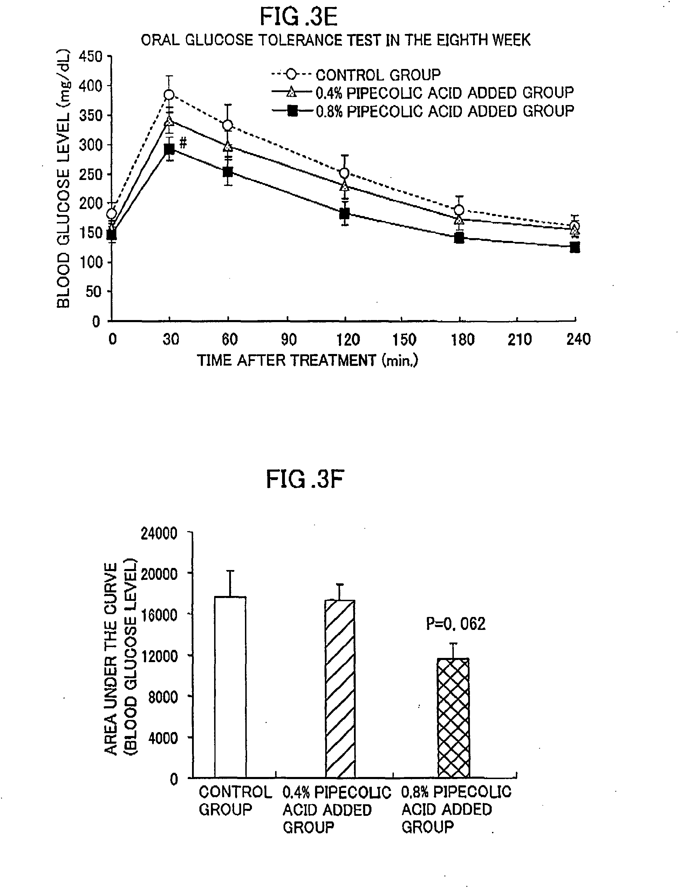

Figure 3E to Figure 3H show diagrams that indicate results of the oral glucose tolerance test conducted in the test 3. Figure 3E to Figure 3H indicate transitions of the blood glucose and insulin concentration after giving glucose at the eighth week and areas under the concentration time curve thereof Each * and # indicates that there is a significant difference as compared with the control group at P<0.05, and P<0.01, respectively. Further, value P in the diagram indicates a risk rate as compared with the control group.

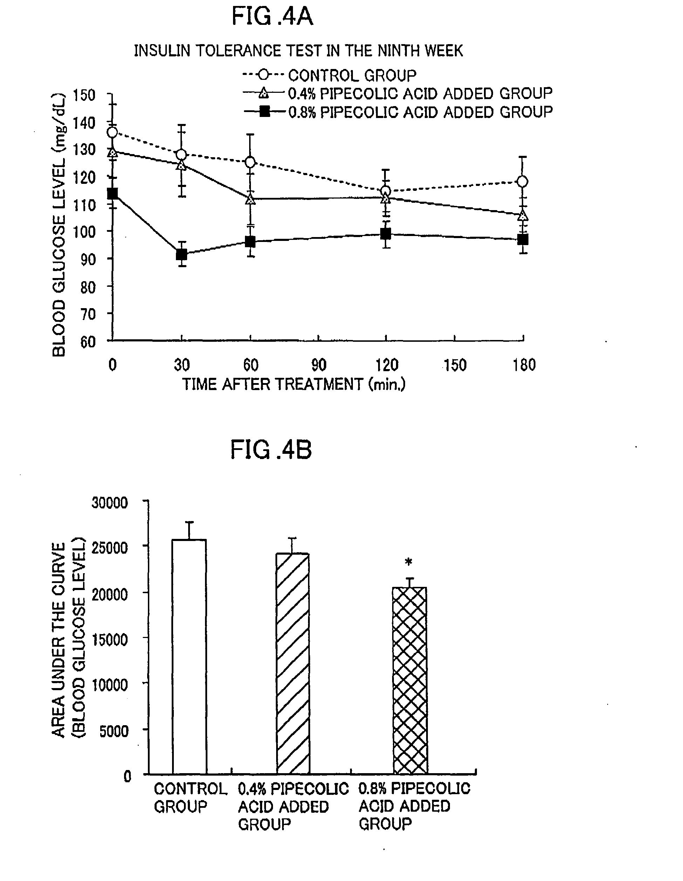

Figure 4 (Fig.4A and Fig.4B) show diagrams that indicate results of the insulin tolerance test conducted in the ninth week of the test 3. 0.5U/kg of body weight of insulin was administered at 0 minute, and it was examined whether the antihyperglycemic action of insulin was enhanced. * indicates that there is a significant difference as compared with the control group, that is, the risk rate P<0.05.

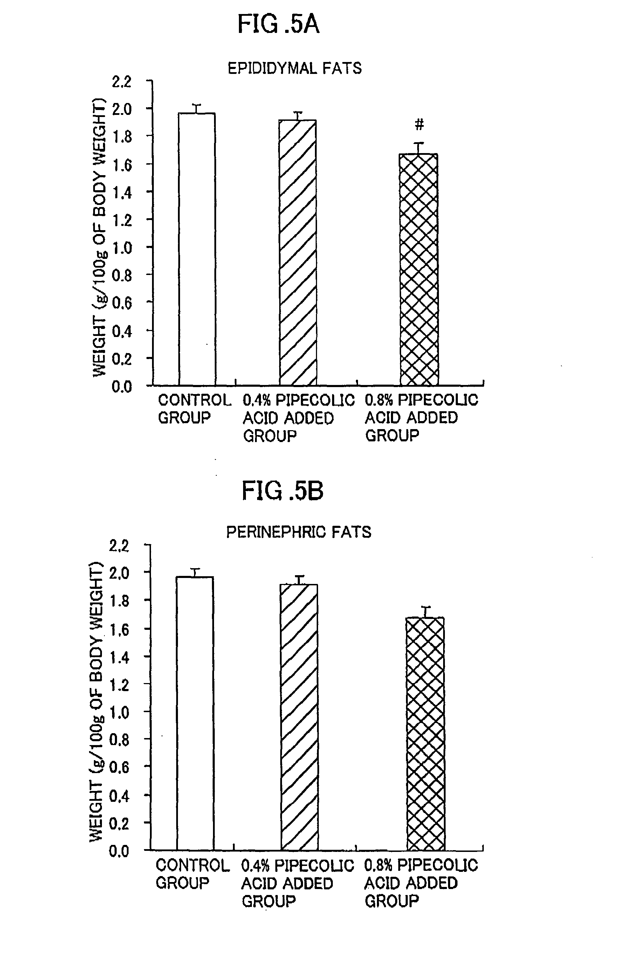

Figure 5 (Fig.5A and Fig.5B) shows weights of various organs in the autopsy of the test 3. Each result indicates the weight per 100g of body weight. Meanwhile, these weights are those of the right side of the body. # indicates that there is a significant difference as compared with the control group, that is, the risk rate P<0.01.

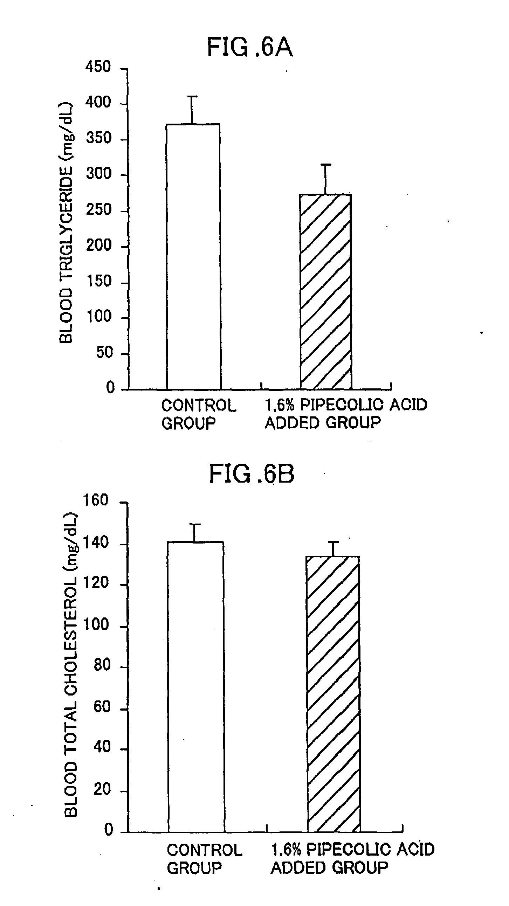

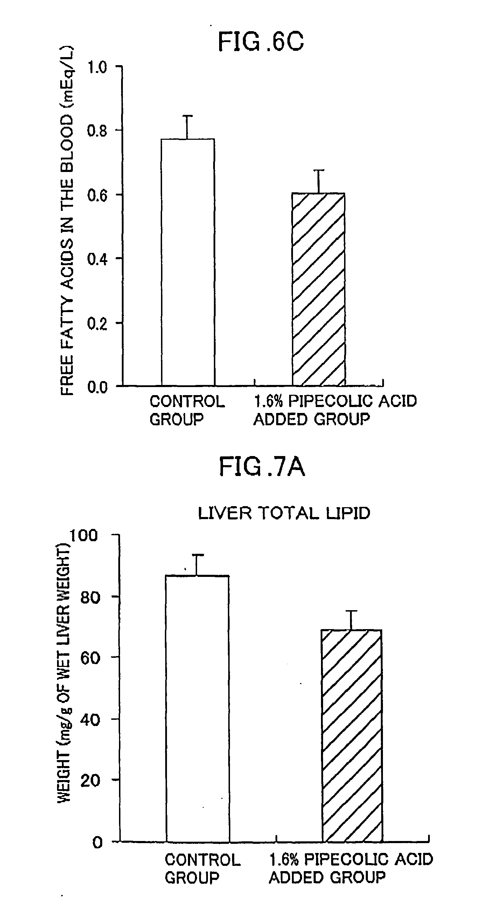

Figure 6 (Fig.6A to Fig.6C) show diagrams of fasting blood triglyceride, blood total cholesterol and free fatty acids in the blood measured in the fifth week after providing pipecolic acid in the test 4.

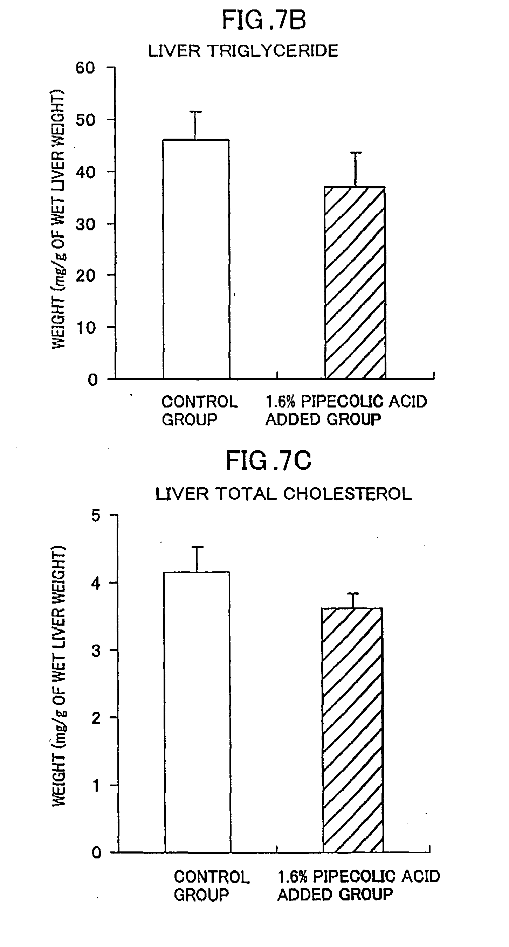

Figure 7 (Fig.7A to 7C) show diagrams of total lipids, triglyceride and total cholesterol in the liver extracted in the fifth week after providing pipecolic acid in the test 4. Value P in the diagram indicates a risk rate as compared with the control group.

Examples of pipecolic acids that can be used in the present invention include L-pipecolic acid, D-pipecolic acid and pipecolic acid derivatives.

The pipecolic acid derivatives used in the present invention means pipecolate betaine itself or compounds that may be the sources of physiologically acceptable pipecolic acid or pipecolate betaine.

The compounds that may be the sources of physiologically acceptable pipecolic acid or pipecolate betaine include the following compounds:
peptides containing pipecolic acids or pipecolate betaine as the component (oligopeptides containing pipecolic acids or pipecolate betaine such as alanyl-pipecolic acid and γ-glutamyl-pipecolic acid);
ester of alkyl pipecolate, alkenyl pipecolate or aralkyl pipecolate;
amide pipecolate;
amide of alkyl pipecolate, alkenyl pipecolate or aralkyl pipecolate;
dialkylamide pipecolate;
N-acyl-pipecolic acid;
alkylester of N-acyl-pipecolic acid;
N-acyl-pipecolic acid amide;
N-acyl-pipecolic acid alkylamide;
N-acyl-pipecolic acid dialkylamide;
ester of pipecolate betaine alkyl, pipecolate betaine alkenyl or pipecolate betaine aralkyl;
pipecolate betaine amide;
amide of pipecolate betaine alkyl, pipecolate betaine alkenyl or pipecolate betaine aralkyl; and
pipecolate betaine dialkylamide.

Here, the carbon number of the alkyl group contained in the aforementioned groups is 1 to 22; that of the alkenyl group is 2 to 22; that of the aralkyl group is 7 to 22; and that of the acyl group is 1 to 22. When the compound has dialkyl, the carbon number of each alkyl group may be the same or different from each other.

Pipecolic acid used in the present invention may be either free forms, salts or solvates. As examples of salts of pipecolic acid in which its carboxyl group forms salts, they include ammonium salts, and salts with alkali metals, e. g. sodium and potassium, salts with alkaline earth metals, e. g. calcium and magnesium, salts with aluminum and zinc, and salts with organic amines, e. g. triethylamine and ethanolamine. As examples of salts of pipecolic acid in which its basic group forms salts, they include those with inorganic acids, e. g. hydrochloric acid, sulfuric acid and phosphoric acid, those with organic carboxylic acids, e. g. acetic acid, citric acid, benzoic acid, maleic acid, fumaric acid, tartaric acid and succinic acid, and those with organosulfonic acids, e. g. methanesulfonic acid and p-toluenesulfonic acid. Examples of the solvates include hydrates and alcohol adducts. Pipecolic acid may be L-form, D-form, DL form or mixture of L-form and D-form in the arbitrary ratio. Of course, two or more kinds of the pipecolic acids mentioned above can be combined.

The content of the pipecolic acid in the composition of the present invention is 20mg/kg to 2000mg/kg of body weight per day in its administered dose and preferably 50mg/kg to 1000mg/kg of body weight per day. When its administered dose per day is less than 20mg/kg of body weight, it might not have desired effect. When the dose is more than 2000mg/kg of body weight, it might have a problem in that it produces toxic effects.

The preferable administration route (intake route) of the composition of the present invention is oral administration, and it is further preferable to take the composition continuously for a few days.

The composition of the present invention can be used for inhibiting increase of the blood glucose level or decreasing the blood glucose level; enhancing insulin sensitivity or improving insulin resistance; inhibiting increase of lipids in the blood or decreasing lipids in the blood; inhibiting accumulation of visceral fats or decreasing accumulated visceral fats; or preventing, diminishing or treating diabetes, arterial sclerosis, obesity or high-blood pressure. Particularly, it can be preferably used for preventing, diminishing or treating arterial sclerosis and arteriosclerosis, obesity, high-blood pressure, or other diabetic complications. Further, it particularly produces excellent effects on inhibiting increase of the blood glucose after meals or improving insulin resistance in type II diabetes. It can also decrease visceral fats or lipids in the blood.

The composition of the present invention can be prepared, in addition to the pharmaceutical composition form, as forms such as foods, health foods, supplements, nutrient compositions or feeds, indicating that they are used for preventing, diminishing or treating the above diseases or pathologies.

In case of the pharmaceutical composition form, the composition of the present invention can be mixed with pharmaceutically acceptable carriers or diluents such as cellulose derivatives, e.g. carboxymethylcellulose and ethyl cellulose; starches, e.g. potato starch and corn starch; sugars, e.g. lactose and sucrose; vegetable oils, e.g. peanut oil, corn oil and sesame-seed oil; polyethylene glycol, alginic acid, gelatin, and talc, and then prepared to dosage forms such as oral agents, e.g. tablets, dispersants, pills, granules, capsules and syrups; injectable solutions, e.g. subcutaneously-injected solutions, intravenously-injected solutions, intramuscularly-injected solutions, injectable solutions for epidural space, and injectable solutions for subarachnoid space; external agents, e.g. intranasal preparation, transdermal preparations, and ointments; suppositories, e.g. rectal suppositories and vaginal suppositories; and intravenous fluid preparations.

The pharmaceutical composition of the present invention can be administered orally or parenterally, e.g., enterally or intravenously.

In case of the food form, optional additives can be used to the composition of the present invention, and then prepared in accordance with ordinary methods. Examples of such additives include products usually used as the components of health foods, such as those for adjusting and improving taste, e.g. fruit juice, dextrin, cyclic oligosaccharide, sugars (fructose, glucose, liquid sugar, sucrose), acidulants, flavoring agents, green powdered tea, and fats and oils; and those for improving texture, e.g. emulsifying agents, collagen, whole powdered milk, thickening agents of polysaccharides, and agar (in case of jelly beverages).

Further, the foods of the present invention can be prepared to the health foods by mixing amino acids, vitamins, egg shell calcium, calcium pantothenate and other minerals, royal jelly, propolis, honey, dietary fibers, Agaricus, chitin, chitosan, capsaicin, polyphenol, carotenoid, fatty acids, mucopolysaccharides, coenzymes, and antioxidants.

In case of the supplement form, the composition of the present invention can be mixed with emulsifying agents, pigments, and flavoring agents and then prepared to the dosage forms such as tablets, capsules and liquids.

The composition of the present invention can also be prepared as feeds for mammals such as swines, bovines, sheep, canines, felines, mice, rats and apes and for avian species such as fowls, pheasants and ostriches. For example, it can be prepared to solid or liquid additives for feeds in accordance with the methods known in the art.

The product forms of the composition of the present invention are not particularly limited and any form is permissible only if usually used amino acids can be taken. Examples of such forms include, in case of oral administration, powders, granules, tablets, liquids (drinks, jelly drinks and the like), candies such as chocolates, wherein a suitable excipient(s) is used, or simple mixture of one or two kinds of the above amino acids. In case of intravenous administration, examples thereof include infusions containing one or two kinds of the above amino acids, water solutions, and ready-for-use amino acid powders.

The following Examples specifically illustrate the present invention. The methods of analysis used herein are a oral glucose tolerance test and a insulin tolerance test. These are the test methods generally used for diagnosing diabetes or insulin resistance and appropriate from the viewpoints of pharmacology and physiology. All results at this time were represented with average value ± standard error. In the statistical analysis, average values among all test groups were tested by using the multiple comparisons of Tukey-Kramer on each measurement item.

### Examples

The following Test Examples further illustrate the present invention. Test Example 1 (Examination of blood glucose changes after injection of glucose when providing normal rats with pipecolic acid for 4 weeks)

### (1) Summary of the test

(a) It was examined whether the increase of the blood glucose after oral injection of glucose to normal rats could be inhibited by providing them with D,L pipecolic acid for 4 weeks.
(b) Male SD rats of 9 weeks old were used to start the experiment.
(c) 24 normal rats were divided into 3 groups (each group consisting of 8 rats) so that their fasting blood glucose and body weights became the same. Feeds adding 0, 0.2 or 0.4% D,L pipecolic acid were provided to each group for 4 weeks. Then, they were fasted for 17 hours, and 10% glucose solution was orally administered to them in its provided amount of 2g/kg of body weight. At 0, 15, 30, 60, and 120 minutes after the administration, the blood glucose level and blood insulin concentration were measured.
(d) In the groups wherein the feeds adding 0.2 or 0.4% D,L pipecolic acid were provided, the increase of the blood glucose after oral injection of glucose was inhibited. Particularly, in the group wherein 0.4% D,L pipecolic acid was provided, the blood glucose of 30 and 60 minutes after the administration was decreased, which is a period of time that the glucose significantly increases. However, the blood insulin concentration did not change in any group and at any time period. (See Figure 1 (Fig.1A to 1C))
(e) From the above results, it was confirmed that pipecolic acid inhibits the increase of the blood glucose level without affecting the blood insulin concentration. It is thought that this happens as a result of enhancement of insulin sensitivity. Therefore, as mentioned in the present invention, it is thought that provision of pipecolic acid has effects on inhibiting the increase of the blood glucose after oral injection of glucose and improving insulin resistance.

### (2) Test details

(a) Constitution of each group is shown in following Table 1.

**Table 1**

| | |
|---|---|
| Test groups | Provided feeds |
| Control group | Commercial feed |
| 0.2% pipecolic acid added group | Commercial feed + 0.2% D,L pipecolic acid |
| 0.4% pipecolic acid added group | Commercial feed + 0.4% D,L pipecolic acid |

(b) Preparation of feeds: D,L pipecolic acid (produced by Aldrich) was mixed in small portions with a commercial feed (CRF-1, produced by Oriental Yeast Co., Ltd.) and further mixed together for 5 minutes by using a universal mixer (produced by Dalton Corporation).
(c) Farming rats: 24 (3 experimental sections × 8) male SD rats of 7 weeks old were purchased from Charles River Laboratories Japan, Inc. and each was separately farmed in an animal room in a light-dark cycle of 12 hours (7:00 to 19:00) at room temperature of 25°C. After habituation for two weeks, they were divided into 3 groups based on their body weights and fasting blood glucoses, and then the above feeds were provided for 4 weeks. Every Mondays, Wednesdays and Fridays during the period, their body weights and amounts of food ingested were measured, and the feeds were added. 4 weeks later, the oral glucose tolerance test was conducted.
(d) Oral glucose tolerance test: Fasting was started at 17:00 of the day before the test. At 10:00 of the test day, 10% glucose solution was orally administered to the rats in its provided amount of 2g/kg of body weight. 0, 15, 30, 60, and 120 minutes after the administration, their blood was collected and their blood glucose levels and blood insulin concentrations were measured. The blood glucose levels were promptly measured after the blood collection by using a biochemical auto-analyzer for animals (Fuji DriChem 5500, produced by Fuji Photo Film Co., Ltd.). Further, the collected blood was centrifuged with a centrifuge (himac CF15D, produced by HitachiKoki Co., Ltd) to take out the blood plasma, and the blood insulin concentrations were measured by using an insulin measurement kit (produced by Morinaga Institute of Biochemical Science, Inc.). The results are shown in Figure 1 (Fig.1A to Fig.1C). Fig. 1A and Fig. 1B indicate the blood glucose transitions and the area under the blood glucose concentration time curve; and Fig. 1C indicates transitions of the blood insulin concentration.

### Test Example 2 (Examination of blood glucose changes after giving glucose when providing obese model rats with pipecolic acid for 4 weeks)

### (1) Summary of the test

(a) It is reported that obese model rats produced by giving high-fat diets express insulin resistance and develop hyperinsulinemia. It was examined whether the increase of the blood glucose and blood insulin concentration after oral injection of glucose to these obese model rats could be inhibited by providing them with D,L pipecolic acid for 4 weeks.
(b) Male SD rats of 6 weeks old were used to start the experiment.
(c) 48 rats were divided into 2 groups so that their fasting blood glucoses and body weights became the same. Normal diets or high-fat diets were given to them for 3 weeks (model production period). After that, each group was further divided into 3 groups (in total: 6 groups) based on their fasting blood glucoses, fasting blood insulin concentrations, and their body weights. Then, feeds wherein 0, 0.2 or 0.4% D,L pipecolic acid were added to normal diets or high-fat diets were provided to the groups for 4 weeks (test period). After providing feeds for 4 weeks, the rats were fasted for 17 hours, and 10% glucose solution was orally administered to them in its provided amount of 2g/kg of body weight. 0, 15, 30, 60, and 120 minutes after the administration, their blood glucose levels and blood insulin concentrations were measured.
(d) It was confirmed that the increase of the blood glucose after oral injection of glucose delayed and the blood insulin concentration became higher in high-fat diet groups as compared with normal diet groups. These results were consistent with the publicly known facts and reports. However, in the groups given feeds wherein D,L pipecolic acid was added to high-fat diets, though the increase of the blood glucose after giving glucose was hardly inhibited, the blood insulin concentration decreased to around the same extent as that of the normal diet groups. (See Figure 2 (Fig.2A to Fig.2D))
(e) Summarizing the above results, pipecolic acid hardly had an effect on inhibiting the increase of the blood glucose of the obese model rats given high-fat diets. On the other hand, pipecolic could significantly decrease the blood insulin concentration. It is thought that this happens because pipecolic acid improved insulin resistance of the obese model rats.

### (2) Test details

(a) Constitution of each group is shown in following Table 2.

**Table 2**

| Test groups | Feeds during model production period | Feeds during the test period |
|---|---|---|
| Normal diet group (no pipecolic acid added) | Normal diets | Normal diets |
| Normal diet + 0.2% pipecolic acid added group | Normal diets | Normal diets + 0.2% D,L pipecolic acid |
| Normal diet + 0.4% pipecolic acid added group | Normal diets | Normal diets + 0.4% D,L pipecolic acid |
| High-fat diet group (no pipecolic acid added) | High-fat diets | High-fat diets |
| High-fat diet + 0.2% pipecolic acid added group | High-fat diets | High-fat diets + 0.2% D,L pipecolic acid |
| High-fat diet + 0.4% pipecolic acid added group | High-fat diets | High-fat diets + 0.4% D,L pipecolic acid |

(b) Test schedule 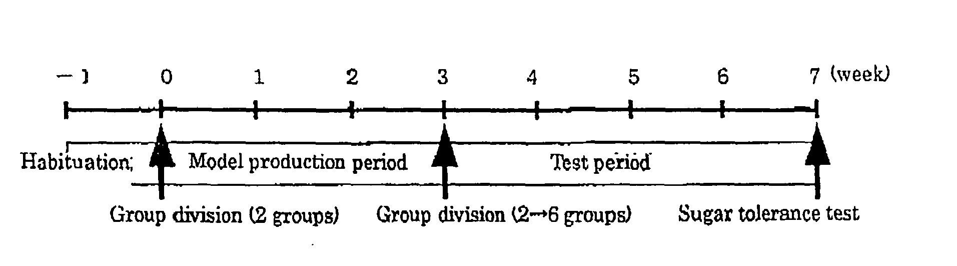 (c) Preparation of feeds: Compositions of normal diets and high-fat diets are shown in following Table 3. 0.2% or 0.4% D,L pipecolic acid (produced by Aldrich) was mixed to these feeds to prepare feeds with normal or high-fat diet + pipecolic acid.

**Table 3**

| | Normal diet | High-fat diet |
|---|---|---|
| Casein | 20.00 | 20.00 |
| L-cystine | 0.30 | 0.30 |
| Cornstarch | 51.75 | 31.75 |
| α -cornstarch | 13.20 | 13.20 |
| Palm oil | 0.00 | 5.00 |
| Soybean oil | 5.00 | 21.00 |
| Cellulose powder | 5.00 | 5.00 |
| AIN93G mineral mixture | 3.50 | 3.50 |
| AIN93G vitamin mixture | 1.00 | 1.00 |
| Choline tartrate | 0.25 | 0.25 |
| t-butylhydroxinon | 0.0014 | 0.0014 |
| Total | 100.00 | 100.00 |

(d) Farming rats: 48 (6 experimental sections × 8) male SD rats of 5 weeks old were purchased from Charles River Laboratories Japan, Inc. and each was separately farmed in an animal room in a light-dark cycle of 12 hours (7:00 to 19:00) at room temperature of 25°C. After naturalizing one week, they were divided into 2 groups based on their body weights and fasting blood glucoses. Then, normal diets were provided to one group and high-fat diets were provided to the other (model production period). After 3 weeks, each group was further divided into 3 groups (in total: 6 groups) so that their body weights, fasting blood glucoses, and fasting blood insulin concentrations became the same. Then, feeds wherein 0, 0.2 or 0.4% D,L pipecolic acid were added to normal diets or high-fat diets were provided to the groups for 4 weeks (test period). Every Mondays, Wednesdays and Fridays during all periods starting from the model production period to the end of the test period, their body weights and amounts of food ingested were measured, and the feeds were added. As of the end of the test period, the sugar tolerance test was conducted.
(e) Oral glucose tolerance test: Fasting was started at 17:00 of the day before the test. At 10:00 of the test day, 10% glucose solution was orally administered to the rats in its provided amount of 2g/kg of body weight. 0, 15, 30, 60, and 120 minutes after the administration, their blood was collected and their blood glucose levels and blood insulin concentrations were measured. The blood glucose levels were promptly measured after the blood collection by using a biochemical auto-analyzer for animals (Fuji DriChem 5500, produced by Fuji Photo Film Co., Ltd.). Further, the collected blood was centrifuged with a centrifuge (himac CF15D, produced by HitachiKoki Co., Ltd) to take out the plasma, and the blood insulin concentrations were measured by using an insulin measurement kit (produced by Morinaga Institute of Biochemical Science, Inc.). The results are shown in Figure 2 (Fig.2A to Fig.2D). (A) indicates the blood glucose transitions and the area under the blood glucose concentration time curve; and (B) indicates transitions of the blood insulin concentration and the area under the blood insulin concentration time curve.

### Test Example 3 (Effects of pipecolic acid on pathologic transitions of model mice with Type II diabetes)

### (1) Summary of the test

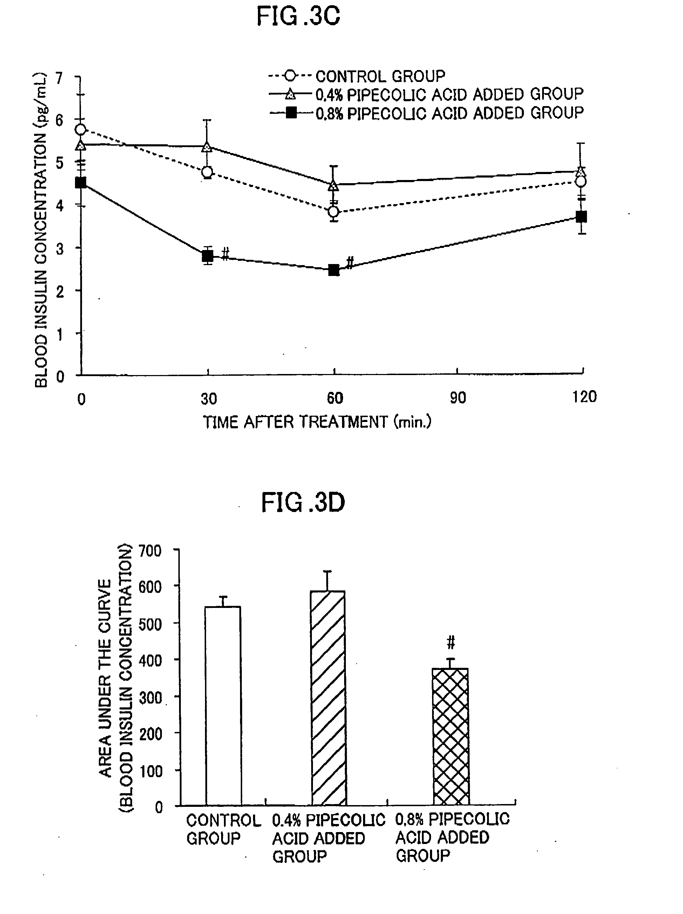

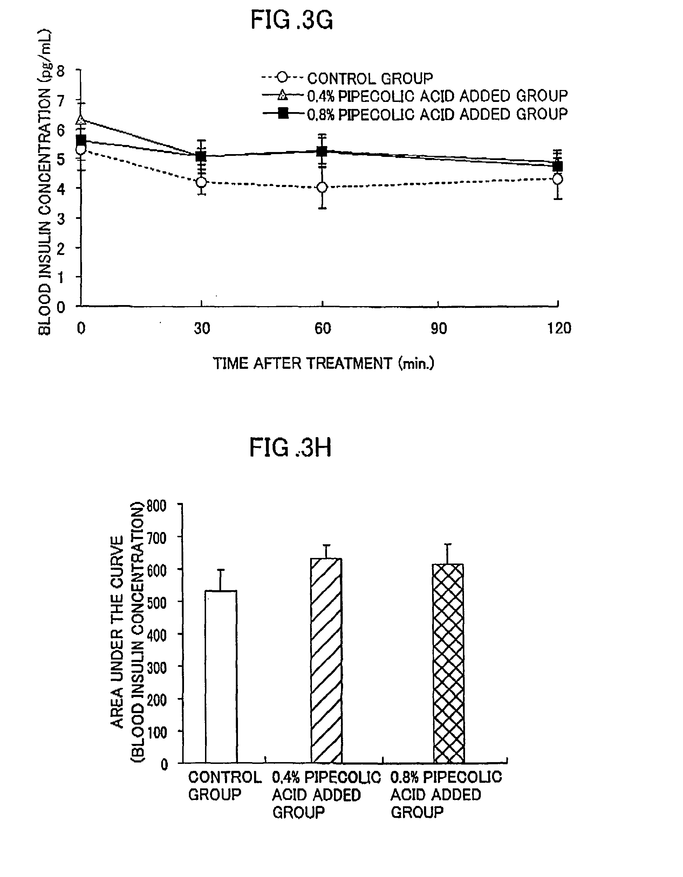

(a) It was examined whether D,L pipecolic acid has an effect of improving the pathology of model mice with Type II diabetes.
(b) Male KK-Ay mice, which are the model mice with Type II diabetes, were used to start the experiment.
(c) 24 KK-Ay mice were divided into 3 groups based on their body weights, and the blood glucose levels and transitions of the blood insulin concentrations when injected glucose. Feeds adding 0, 0.4 or 0.8% D,L pipecolic acid were provided to each group for 10 weeks. At the second and eighth weeks after starting the test, their blood glucose levels and blood insulin concentrations after oral injection of glucose were measured. At the ninth week, the insulin tolerance test was conducted, and at the tenth week, autopsy was conducted to the samples to measure weights of epididymal fats and perinephric fats.
(d) In the group wherein 0.4% D,L pipecolic acid was added, the increase of their blood glucoses was inhibited as compared with those of the additive-free group in the oral glucose tolerance test of the second week. However, no strong effect of inhibiting the increase of the blood glucose was confirmed at the eighth week. On the other hand, in the group wherein 0.8% D,L pipecolic acid was added, the effect of inhibiting the increase of the blood glucose after injection of glucose was seen until the eighth week. (See Figure 3 (Fig.3A to Fig.3H))
   Besides, in the insulin tolerance test conducted at the ninth week, decrease of the blood glucose after administering insulin was promoted in the group wherein 0.8% D,L pipecolic acid was added, as compared with that of the additive-free group. (See Figure 4A and Figure 4B) It was seen that weights of epididymal fats and perinephric fats collected in the autopsy were decreased by adding 0.8% D,L pipecolic acid. (See Figure 5A and Figure 5B)
(e) Since pipecolic acid (particularly, 0.8% addition thereof) showed the results that it improved diabetic pathologies in the oral glucose tolerance test and insulin tolerance test, it is indicated that it is effective in preventing and/or treating Type II diabetes. Further, since accumulation of epididymal fats or perinephric fats, which are visceral fats, becomes one of the risk factors inducing arterial sclerosis as well as the cause of obesity, it is thought that pipecolic acid that decreased the weight of those fats also has an anti-obese effect and antiatherogenic effect.

### (2) Test details

(a) Constitution of each group is shown in following Table 4.

**Table 4**

| Test groups | Strain (n: number) | Provided feeds |
|---|---|---|
| Control group (no pipecolic acid | KK-Ay (n = 8) | Commercial feed |
| 0.4% pipecolic acid added group | KK-Ay (n = 8) | added) Commercial feed + 0.4% D,L pipecolic acid |
| 0.8% pipecolic acid added group | KK-Ay (n = 8) | Commercial feed + 0,8% D,L pipecolic acid |

(b) Preparation of feeds: D,L pipecolic acid (produced by Aldrich) was mixed in small portions with a commercial feed (CRF-1, produced by Oriental Yeast Co., Ltd.) and further mixed together for 5 minutes by using a universal mixer (produced by Dalton Corporation).
(c) Farming mice: 24 male KK-Ay mice of 5 weeks old were purchased from Clea Japan, Inc. and each was separately farmed in an animal room in a light-dark cycle of 12 hours (7:00 to 19:00) at room temperature of 25°C. Then, KK-Ay mice were divided into 3 groups based on their blood glucose levels and blood insulin concentrations measured when giving glucose. After habituation for two weeks, the above feeds were provided to them for 10 weeks. At the second and eighth weeks, the oral glucose tolerance test was conducted, and their blood glucoses and blood insulin concentrations after oral injection of glucose were measured. At the ninth week, the insulin tolerance test was conducted, and insulin resistance of each group was examined. Then, at the tenth week, autopsy was conducted to the samples, and weights of epididymal fats and perinephric fats were measured to see effects of pipecolic acid on weights of visceral fats.
(d) Oral glucose tolerance test: Fasting was started at 20:00 of the day before the test. At 10:00 of the test day, 10% glucose solution was orally administered to the mice in its provided amount of 1g/kg of body weight. 0, 30, 60, 120, 180, and 240 minutes after the administration, their blood was collected and their blood glucose levels and blood insulin concentrations were measured. The blood glucose levels were promptly measured after the blood collection by using a biochemical auto-analyzer for animals (Fuji DriChem 5500, produced by Fuji Photo Film Co., Ltd.). Further, the collected blood was centrifuged with a centrifuge (himac CF15D, produced by HitachiKoki Co., Ltd) to take out the plasma, and the blood insulin concentrations were measured by using an insulin measurement kit (produced by Morinaga Institute of Biochemical Science, Inc.). The results are shown in Figure 3 (Fig.3A to Fig.3H). Fig.3A to Fig.3D indicate the second week's and Fig.3E to Fig.3H indicate the eighth week's transitions of the blood glucose and insulin concentration and areas under the concentration time curve thereof.
(e) Insulin tolerance test: Fasting was started at 18:00 of the day before the test. At 10:00 of the test day, Insulin was subcutaneously administered to the mice in its provided amount of 0.5U/kg of body weight. 0, 30, 60, 120 and 180 minutes after the administration, their blood was collected and their blood glucose levels were measured by using a biochemical auto-analyzer for animals (Fuji DriChem 5500, produced by Fuji Photo Film Co., Ltd.). The results are shown in Figure 4A and Figure 4B.
(f) Autopsy: Fasting was started at 18:00 of the day before. Starting at 10:00 of the next day, autopsy was conducted to extract epididymal fats and perinephric fats and measure their wet weights. The results are shown in Figure 5A and Figure 5B.

### Test Example 4 (Effects of pipecolic acid on blood and liver lipid concentrations of model mice with Type II diabetes)

### (1) Summary of the test

(a) It was examined whether D,L pipecolic acid has an effect on blood and liver lipids of model mice with Type II diabetes.
(b) Male KK-AY mice of 6 weeks old were used to start the experiment.
(c) 16 KK-Ay mice were divided into 2 groups based on their body weights. Feeds wherein 0 or 1.6% D,L pipecolic acid was added were provided to each group for 5 weeks. At the fifth week, they were fasted, and their blood was collected and their livers were extracted to measure their fasting blood triglyceride, fasting blood total cholesterol, fasting free fatty acids in the blood, total lipids in the liver, liver triglyceride and liver total cholesterol.
(d) 1.6% D,L pipecolic acid added group decreased blood triglyceride and free fatty acids in the blood, liver total cholesterol, and liver triglyceride by about 20% as the average value as compared with the control group. (See Figures 6A. to Figure 6C and Figure 7A, to Figure 7C) As for the total lipids in the liver, it was confirmed that they significantly correlate with the additive amount of pipecolic acid.
(e) Pipecolic acid has a high possibility to have an effect of enhancing insulin sensitivity because, in this test, pipecolic acid decreased lipids in the liver and free fatty acids in the blood, which are known as the factors inducing insulin resistance. In addition to it, since addition of pipecolic acid to feeds decreased blood triglyceride that is a risk factor of arterial sclerosis, it is thinkable, combining its effect of inhibiting accumulation of visceral fats in the above test, that it has multiple effects of treating and/or preventing arterial sclerosis. Further, because arterial sclerosis is one of the causes inducing high-blood pressure, it is indicated that pipecolic acid is effective in treating and/or preventing high-blood pressure.

### (2) Test details

(a) Constitution of each group is shown in following Table 5.

**Table 5**

| Test groups | Provided feeds |
|---|---|
| Control group (no pipecolic acid added) | Commercial feed |
| 1.6% pipecolic acid added group | Commercial feed + 1.6% D,L pipecolic acid |

(b) Preparation of feeds: D,L pipecolic acid (produced by Aldrich) was mixed in small portions with a commercial feed (CRF-1, produced by Oriental Yeast Co., Ltd.) and further mixed together for 5 minutes by using a universal mixer (produced by Dalton Corporation).
(c) Farming mice: 16 (2 experimental sections x 8) male KK-Ay mice of 4 weeks old were purchased from Clea Japan, Inc. and each was separately farmed in an animal room in a light-dark cycle of 12 hours (7:00 to 19:00) at room temperature of 25°C. After naturalizing two weeks, they were divided into 2 groups based on their body weights, and the above feeds were provided to them for 5 weeks. Every Mondays, Wednesdays and Fridays during the period, their body weights and amounts of food ingested were measured, and the feeds were added. After 5 weeks passed, they were fasted for 17 hours, their blood was collected and their livers were extracted. The fasting blood triglyceride, fasting blood total cholesterol, fasting free fatty acids in the blood, total lipids in the liver, liver triglyceride and liver total cholesterol were measured by using those samples.
(d) Measurement of lipids in the blood: The collected blood was centrifuged with a centrifuge (himac CF15D, produced by HitachiKoki Co., Ltd) and the obtained blood plasma was used as a sample. Blood triglyceride and blood total cholesterol were measured by using a biochemical auto-analyzer for animals (Fuji DriChem 5500, produced by Fuji Photo Film Co., Ltd.). Further, free fatty acids in the blood were measured by using a NEFA E-test Wako (produced by Wako Pure Chemical Industries, Ltd.). The results are shown in Figure 6A to Figure 6C.
(e) Measurement of lipids in the liver: About 0.5g of a liver sample is homogenized in 10mL of chloroform/methanol (2:1) solution, and lipids are extracted overnight. The next day, the liver residue is removed by a Kiriyama funnel. Then, the obtained substance is filled up to 10mL with a chloroform/methanol solution. 2mL of normal saline solution is added thereto and stirred for 10 minutes by a shaker (model YS·8D, produced by Yayoi Co., Ltd.). Then, the mixture is left at rest for 2 or more hours, a supernatant thereof is sucked, and then filled up to 10mL with a chloroform/methanol solution. 5mL thereof is concentrated by drying with a centrifugal evaporator, and its weight measured becomes total lipids in the liver.

Liver triglyceride and total cholesterol were measured by drying 40 µ L of the above lipid extracted solution; remelting the substance with isopropanol containing 1% Triton X-100; and using Triglyceride E-test Wako and Free cholesterol E-test Wako (produced by Wako Pure Chemical Industries, Ltd.). The results are shown in Figure 7A to Figure 7C.

## Claims

1. A composition containing pipecolic acid in its administered dose of 20mg/kg to 2000mg/kg of body weight per day.

2. The composition according to claim 1, wherein the administered dose of pipecolic acid is 50mg/kg to 1000mg/kg of body weight per day.

3. The composition according to claim 1 or 2, wherein pipecolic acid is selected from the group consisting of L-pipecolic acid, D-pipecolic acid and L- or D-pipecolic acid derivatives.

4. A composition containing pipecolic acid for inhibiting increase of the blood glucose level or decreasing the blood glucose level; enhancing insulin sensitivity or improving insulin resistance; inhibiting increase of lipids in the blood or decreasing lipids in the blood; inhibiting accumulation of visceral fats or decreasing accumulated visceral fats; or preventing, diminishing or treating diabetes, arterial sclerosis, obesity or high-blood pressure.

5. A pharmaceutical composition containing the composition according to any one of claims 1 to 4.

6. A food containing the composition according to any one of claims 1 to 4.

7. A supplement containing the composition according to any one of claims 1 to 4.

8. A feed containing the composition according to any one of claims 1 to 4.

9. Use of pipecolic acid in the manufacture of a therapeutic agent for inhibiting increase of the blood glucose level or decreasing the blood glucose level; enhancing insulin sensitivity or improving insulin resistance; inhibiting increase of lipids in the blood or decreasing lipids in the blood; inhibiting accumulation of visceral fats or decreasing accumulated visceral fats; or preventing, diminishing or treating diabetes, arterial sclerosis, obesity or high-blood pressure.

10. A use according to claim 9, wherein the therapeutic agent is arranged in a dosage form which provides from 20mg to 2000mg of pipecolic acid per kg body weight per day.

11. A use according to claim 9 or claim 10, wherein the therapeutic agent is a pharmaceutical composition, food, feed or supplement.
